Europäisches Patentamt

European Patent Office

Office européen des brevets

(11) Publication number: **0 173 297**
A2

# (12) EUROPEAN PATENT APPLICATION

(21) Application number: **85110744.1**

(22) Date of filing: **27.08.85**

(51) Int. Cl.⁴: **C 07 D 473/12,** A 23 F 5/22

(30) Priority: **28.08.84 US 644957**

(43) Date of publication of application: **05.03.86**
**Bulletin 86/10**

(84) Designated Contracting States: **AT BE CH DE FR GB IT LI LU NL SE**

(71) Applicant: **W.R. GRACE & CO., Grace Plaza 1114 Avenue of the Americas, New York New York 10036 (US)**

(72) Inventor: **Welsh, William Alan, 12550 Hall Shop Road, Fulton Maryland 20759 (US)**

(74) Representative: **UEXKÜLL & STOLBERG Patentanwälte, Beselerstrasse 4, D-2000 Hamburg 52 (DE)**

(54) Caffeine adsorption on amorphous silica.

(57) Adsorbents comprising amorphous silicas, specifically silica gels and precipitated silicas, are useful in processes for the removal of caffeine from solutions. Dehydroxylated, hydrophobic silicas are preferred for use with aqueous solutions, for use with non-aqueous solutions hydrophilic silicas are preferred. The adsorbed caffeine may be eluted from the silica with water.

EP 0 173 297 A2

## BACKGROUND OF THE INVENTION

This invention relates to a method for removing caffeine from solutions by contacting the solutions with a caffeine adsorbent. More specifically, it has been found that amorphous silicas are particularly well suited for use as adsorbents for caffeine and can be used to adsorb caffeine from both aqueous and non-aqueous solutions. After removal of the caffeine-poor solution, the caffeine can be eluted from the silica with water. The regenerated silica adsorbent can be recycled for further use.

Caffeine, an alkaloid which occurs free and bound in such plants as coffee, tea, cacao and kola nuts, is the focus of substantial commercial interest. On the one hand, new and efficient methods for the decaffeination of coffee and tea extracts are pursued to meet the demand for coffee and tea flavored beverages which do not stimulate the central nervous system as do caffeinated products. On the other hand, the extracted and purified caffeine is added to many carbonated beverages precisely for its stimulant effect and also is used in many pharmaceuticals, either alone or in conjunction with other ingredients.

The description herein will address the decaffeination of coffee, but it should be understood that this is by way of example and the invention described is applicable for the removal of caffeine from any solution, regardless of its origin. Decaffeination of coffee may be by solvent or water extraction from green or roasted coffee beans. Methylene chloride is the solvent of choice in solvent extraction processes. Where caffeine is extracted from the beans in a water extraction process, the extract is then contacted with a solvent, e.g., methylene chloride, to selectively extract the caffeine from the aqueous solution. Following either process, the caffeine must be separated from the solvent and must be purified to meet

- 2 -

食

0 173 297

food or pharmaceutical grade specifications, as desired.

Numerous materials have been suggested for the selective adsorption of caffeine from solution. For example, U. S. 4,113,888 (Henig et al.) discloses the use of an adsorption medium such as macroreticular acrylic and/or styrene and/or divinylbenzene adsorption resins in an adsorption process for decaffeinating aqueous extracts of green or roasted coffee, and U. S. 4,324,840 (Katz) discloses use of a solid caffeine adsorbent which is made more selective to caffeine by a thin layer of a water-immiscible, caffeine-specific solvent. U. S. 4,331,694 (Izod) discloses selective adsorption of caffeine using a particular modified form of zeolite Y as the adsorbent. In discussing prior proposed decaffeination processes, Izod states that a variety of adsorbent materials have been proposed, including ion exchange resins, activated carbons and a number of silicate minerals such as clays, hydrated magnesium trisilicate and hydrated aluminum silicate, but that these involve serious drawbacks, attributable either to relatively poor selectivity and/or capacity of the adsorbent for the caffeine.

## SUMMARY OF THE INVENTION

Caffeine can be effectively removed from aqueous or non-aqueous solutions by adsorption onto amorphous silica. The caffeine then is desorbed by eluting with water, after which the caffeine may be purified and crystallized by conventional methods. Following elution of the adsorbed caffeine, the regenerated silica adsorbent may be recycled for further use in this process.

It is a primary purpose of this invention to provide a method for removing caffeine from solution by utilizing an adsorbent for the caffeine.

- 3 -

It is a related purpose to provide such a method in which the caffeine can be desorbed by water elution, eliminating the need for the introduction or use of further solvents, and reducing the number of process steps required to produce food or pharmaceutical grade caffeine.

In addition, it is intended to teach the use of a caffeine adsorbent which has the versatility to be used with a variety of commercial caffeine extraction systems, whether aqueous or non-aqueous.

A further object of this invention is to provide a cyclical caffeine removal process whereby the amorphous silica adsorbent is regenerated by the elution of adsorbed caffeine and may be reused in additional adsorption cycles.

DETAILED DESCRIPTION OF THE INVENTION

It has been found that amorphous silicas are particularly well suited for removing caffeine from solutions by adsorption. The process for the bulk removal of caffeine from solution, as described in detail herein, essentially comprises the steps of selecting a solution comprising caffeine, selecting an adsorbent comprising amorphous silica, contacting the solution and the adsorbent, allowing the caffeine to be adsorbed and separating the resulting caffeine-enriched adsorbent from the caffeine-depleted solution. Since the competitive adsorption of water onto the silicas in aqueous solutions generally displaces other adsorbates, it can be seen that desorption of the caffeine easily can be accomplished by eluting with water. This water elution step also serves to regenerate the silica adsorbent so that it can be recycled. The caffeine-adsorption abilities of amorphous silicas can be demonstrated in both aqueous and non-aqueous systems. Dehydroxylated, hydrophobic silicas are preferred for use in aqueous systems, while hydroxylated, hydrophilic silicas are preferred for use in non-aqueous systems.

- 4 -

The term "amorphous silica" as used herein is intended to embrace high surface area silica gels and precipitated silicas in their various prepared or activated forms. Both silica gels and precipitated silicas are prepared by the destabilization of aqueous silicate solutions by acid neutralization. In the preparation of silica gel, a silica hydrogel is formed which then typically is washed and dried so that the resulting gel is substantially free of water. Although the silica hydrogel will adsorb some caffeine if used in this method, for a commercially suitable adsorption process only the dried silica gels or precipitated silicas should be used. In the preparation of precipitated silicas, the destabilization is carried out in the presence of polymerization inhibitors, such as inorganic salts, which cause precipitation of hydrated silica. The precipitate typically is filtered, washed and dried.

By "high surface area" it is intended that the amorphous silica used in this decaffeination process be characterized by significant internal porosity, that is, by significantly more than the expected geometric surface area. For example, silicas exhibiting greater than about 50 square meters per gram surface area would be considered to have high surface areas as that term is used herein.

The basic physical properties of the main types of silica gels and precipitated silicas are shown in Table I. The information regarding these properties was obtained from Kirk-Othmer, Encyclopedia of Chemical Technology, Vol. 18, pp 64,66 (2nd rev. ed. 1969). It is expected that amorphous silicas as defined above will be suitable adsorbents for the removal of caffeine from solutions in the manner described herein. However, it can be seen by reference to Table I that the physical

- 5 -

properties of these amorphous silicas vary along a
continuum.  The silicas described in Table I indicate the
most commonly available commercial silicas on that
continuum.

TABLE I

| Silica Gels: | Bulk Density[1] | Surface Area[2] | Average Pore Diam. |
|---|---|---|---|
| RD | 0.67-0.75 | 750-800 | 22-26 |
| ID | 0.35-0.40 | 300-350 | 120-160 |
| LD | 0.12-0.17 | 100-200 | 180-220 |

Precipitated Silicas:

| | Bulk Density[1] | Surface Area[2] | |
|---|---|---|---|
| | $4-10^4$ | 200-400 | |
| | $15^4$ | 140-160 | |

---

1 - Grams per milliliter

2 - Square meters per gram

3 - Angstroms

4 - Pounds per cubic foot

In the preferred embodiment of this invention, the
silica adsorbent will have the highest possible surface
area in pores which are large enough to permit access to
the caffeine molecules, while being capable of maintaining
good structural integrity upon contact with an aqueous
media.  The requirement of structural integrity is particularly important where the silica adsorbents are used in continuous flow systems, which are susceptible to disruption and plugging. Amorphous silicas for use in this decaffeination process preferably should have a surface area of about 50 to 900 square meters per gram and an average pore diameter of about 60 to about 300 Angstroms.

Regular density ("RD") silica gels, which have extremely fine pores (i.e., less than about 50 Angstroms) and which will shatter when contacted by water, normally will not be desirable of this method, even though they are very high surface area silicas. However, chemically or physically modified regular density silica gels or silica co-gels which have improved structural integrity and are resistant to shattering may be used in this method if desired. For example, silica co-gels containing at least minor amounts of materials such as alumina, titania, zirconia and the like may be used.

Low density ("LD") silica gels or aerogels may be used to adsorb caffeine from solutions, but are not preferred because of process difficulties which may be encountered. Since LD silica gels have a lower surface area, the adsorption efficiency will be somewhat lower than for high surface area silicas.

The preferred embodiment of this invention utilizes a high surface area, small pore size amorphous silica or modified amorphous silica with good structural integrity. The physical properties of intermediate density ("ID") silica gels reflect this desired balance of characteristics and the ID silica gels are particularly well suited for method of caffeine adsorption described herein. For example, it is preferred to use a silica with a surface area of about 300 to about 350 square meters per gram and with an average pore volume of about 120 to 160 Angstroms.

- 7 -

Comparable properties are found in precipitated silicas. For example, suitable precipitated silicas are commercially available with surface areas of up to about 500 square meters per gram; precipitated silicas with higher surface areas also may be used. The other criteria described for selection of suitable silica gels are applicable for selection of suitable precipitated silicas for use in this invention.

The purity of the amorphous silica used in this invention is not believed to be critical in terms of the adsorption of caffeine. However, the end product(s) of most, if not all, systems employing decaffeination processes will be food or pharmaceutical grade material(s). Therefore, care preferably should be taken to ensure that the silica used does not contain leachable impurities which could compromise the desired purity of the product(s). It is preferred, therefore, to use a substantially pure amorphous silica. For example, a preferred ID silica gel would contain about 99.68 wt.% silica as $SiO_2$, about 0.01 wt.% iron as $Fe_2O_3$, about 0.10 wt.% aluminum as $Al_2O_3$, about 0.02 wt.% titanium as $TiO_2$ about 0.07 wt.% calcium as CaO, about 0.06 wt.% sodium as $Na_2O$, about 0.03 wt.% zirconium as $ZiO_2$, and about 0.03 wt.% trace elements.

The solution from which the caffeine is adsorbed may be aqueous or non-aqueous. Non-aqueous solvents for caffeine such as chlorinated hydrocarbons, preferably methylene chloride, may be used. The adsorption method of this invention is suitable for use with a pure or substantially pure caffeine solution, or with a more complex solution which comprises caffeine, such as a tea or coffee bean extract. Extracts of either green or roasted coffee beans will be suitable. It is known, however, that other compounds present in extracts (e.g., chlorogenic acid) complex with caffeine and may tend to interfere to some degree with caffeine adsorption.

Aqueous or non-aqueous extracts may be contacted directly with the amorphous silica adsorbent for removal of caffeine. Alternatively, the caffeine first may be removed from the extract with carbon dioxide under supercritical conditions of temperature and pressure as described in U.S. 4,348,422 (Zosel), next removing the caffeine from the resulting carbon dioxide-water-caffeine phase by the decaffeination method of this invention.

The preferred method of activation of the silica absorbent will differ depending on whether the caffeine solution to be contacted is aqueous or non-aqueous. For use with aqueous solutions, more hydrophobic silicas are the most effective. The selected amorphous silica therefore should be pretreated to dry and dehydroxylate the surface. For example, the silica may be activated by calcining in dry air at temperatures of about 500 to 900 C as indicated in Iler, The Chemistry of Silica, p.635 (1980).

When adsorption is from a non-aqueous solution, a more hydrophilic silica surface will provide the most effective caffeine adsorbent. The amorphous silica should be prepared according to standard activation procedures, which result in a relatively more hydroxylated, hydrophilic surface, substantially free of adsorbed water. For example, heating to about 100 to 500°C in dry air or heating to lower temperatures in a vacuum will activate the silica while allowing its surface to remain hydroxylated.

The adsorption step itself is accomplished by conventional process steps in which the silica and the caffeine solution are contacted, preferably in a manner which facilitates the adsorption. The adsorption step may be by any convenient batch or continuous process, although for efficiency and economy a continuous flow packed bed process is preferable. A multiple column configuration is most preferred, particularly when processing an aqueous caffeine solution since the efficiency of the silicas in adsorbing caffeine from aqueous solutions is somewhat lower than when adsorbing from non-aqueous solutions. In a batch system, agitation or other mixing will enhance the adsorption efficiency of the silica.

Following the adsorption step, the caffeine-depleted solution is removed, i.e., by draining. The adsorbed caffeine is removed from the silica by eluting with water or a dilute aqueous solution at the desired temperature. Particularly where adsorption is from an aqueous solution, the process will be more efficient if the adsorption is conducted at a relatively lower temperature followed by elution with relatively warmer water; although elevated elution temperatures may be desired in any event. The caffeine-rich aqueous eluate may be subjected to additional finishing processes, if desired, such as further purification and/or crystallization. The capacity of the silica to adsorb caffeine is regenerated by elution and subsequent drying where appropriate. The silica then may be recycled for contact with fresh caffeine solutions.

The examples which follow are given for illustrative purposes and are not meant to limit the invention described herein. The following abbreviations have been used throughout in describing the invention:

DI   - deionized
diam - diameter
gm   - gram(s)
ID   - intermediate density

LD   — low density

m    — meter

ml   — milliliter(s)

%    — percent

RD   — regular density

RH   — relative humidity

wt   — weight


## Example I
### (Preparation of Silica Gels)

Two silica gels, one regular density ("RD") and one intermediate density ("ID"), were obtained from the Davison Chemical Division of W. R. Grace & Co. Samples of each type of gel were activated in two ways. The first set of samples underwent standard activation at 230°C. The second set of samples was activated by calcining in dry air for two hours at 705°C. The properties of each silica gel, (after activation) are shown in Table II. Also shown on Table II are the properties of VN-3, a typical commercial precipitated silica, obtained from the Chemicals Division of Degussa, Inc.

### TABLE II

|                                  | RD   | ID    | RD(705°C) | ID(705°C) | VN-3    |
|----------------------------------|------|-------|-----------|-----------|---------|
| Total Volatiles (wt%)            | 5.5  | 2.34  | 1.83      | 0.49      | 9.08    |
| Surface Area ($m^2$/gm)          | 815  | 341   | 506       | 311       | 206     |
| Water Adsorption (wt% at 10% RH) | 8.6  | 3.02  | 0.80      | 0.03      | - -     |
| Particle Size (standard mesh)    | 4x8  | 16x40 | 4x8       | 16x40     | 19.8[1] |

---

[1] Average particle size of VN-3 in microns.

Total volatiles were measured by weighing a sample of each material, heating it at 955°C for one hour and reweighing the sample to determine the difference. Surface area, indicated as square meters per gram, was determined by the Brunauer-Emmett-Teller method (B-E-T) (Brunauer et al., J. Am. Chem. Soc., Vol. 60, page 309 (1938)), using nitrogen as the adsorbate. Water adsorption was determined by measuring the weight difference of the silica on exposure to 10% relative humidity at 25° until equilibrium is achieved, that is, until no further weight increase is observed. A substantial decrease, at a comparable surface area, in water adsorption at low relative humidity is indicative of the dehydroxylation and hydrophobicity of the calcined samples. Particle size is indicated by standard mesh designations or by microns, as shown.

The silica hydrogel precursor to the RD silica gel was obtained for use in Example III. This hydrogel, Graded Silica Gel Code 52, was obtained from the Davison Chemical Division of W. R. Grace & Co.

## Example II
### (Aqueous Solution)

An aqueous solution of caffeine was prepared by dissolving 1.5 gm caffeine in 100 ml DI water. Samples (2.5 gm) of the RD, ID and ID (705°C) silicas prepared in Example I were placed in 25 ml aliquots of the caffeine solution and allowed to sit at ambient conditions overnight. A blank was prepared with 25 ml caffeine solution with no adsorbent and also was left overnight. The solutions were sampled and were analyzed for caffeine by liquid chromatography. The results, shown in Table III, indicate that the silicas have capacity for the adsorption of caffeine from aqueous solution and that the more hydrophobic silicas, RD(705°C) and ID(705°C), are somewhat more effective in the adsorption of caffeine from an aqueous solution.

TABLE III

| Adsorbent | Relative Solution Caffeine Content | Wt.% Caffeine On Adsorbent |
|---|---|---|
| Blank | 1.00 | - - |
| ID | 0.81 | 2.85 |
| ID(705°C) | 0.75 | 3.75 |
| RD(705°C) | 0.73 | 4.05 |

## Example III
### (Non-Aqueous Solution)

A non-aqueous solution of caffeine was prepared by dissolving 1.5 gm caffeine in 100 ml of methylene chloride. Samples (2.5 gm) of the silicas prepared in Example I were placed in 25 ml aliquots of the caffeine solution and allowed to sit in closed containers at ambient conditions overnight. In preparing the hydrogel sample, 2.5 gm on a silica basis of the hydrogel was placed in the caffeine solution. A blank was prepared with 25 ml caffeine solution with no adsorbent and also was left overnight. The solutions were sampled and analyzed for caffeine by liquid chromatography.

The results, shown in Table III, indicate that the silicas have capacity for the adsorption of caffeine from non-aqueous solution and that the more hydrophilic silica gels (RD and ID) are substantially more effective in the adsorption of caffeine from a methylene chloride solution. The precipitated silica (VN-3) and the more hydrophobic silica gels (RD(703°C) and ID (703°C)) show approximately equivalent adsorption. The hydrogel adsorbed only a minor amount of caffeine.

## Table IV

| Adsorbent | Relative Solution Caffeine Content | Wt% Caffeine On Adsorbent |
|-----------|-----------------------------------|---------------------------|
| Blank | 1.00 | - - |
| ID | 0.31 | 10.4 |
| RD | 0.09 | 13.7 |
| ID (705°C) | 0.54 | 6.9 |
| RD (705°C) | 0.60 | 6.0 |
| VN-3 | 0.58 | 6.3 |
| Hydrogel | 0.92 | 1.2 |

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

0 173 297

WHAT WE CLAIM IS:

1. A process for the bulk removal of caffeine from solution by absorbing said caffeine onto amorphous silica, comprising:
   a)  selecting a solution comprising caffeine,
   b)  selecting an adsorbent comprising amorphous silica,
   c)  contacting the solution of step (a) and the adsorbent of step (b),
   d)  alllowing caffeine from said solution to be adsorbed onto said adsorbent, and
   e)  separating the resulting caffeine-enriched adsorbent from the caffeine-depleted solution.

2. The process of claim 1 in which the adsorbed caffeine is removed from the caffeine-enriched adsorbent of step (e) by eluting with water.

3. The process of claim 1 in which said solution of step (a) is an aqueous solution and said amorphous silica of step (b) is hydrophobic.

4. The process of claim 3 in which said amorphous silica has been activated in a manner which causes dehydroxylation of the silica surface.

5. The process of claim 1 in which said solution of step (a) is a non-aqueous solution and said amorphous silica of step (b) is hydrophilic.

6. The process of claim 5 in which said amorphous silica has been activated in a manner which allows the silica surface to remain hydroxylated.

7. The process of claim 1 in which said solution comprises methylene chloride or comprises carbon dioxide under supercritical conditions of temperature and pressure.

8. The process of claim 1 in which said amorphous silica has a surface area of at least about 50 $m^2/g$.

9. The process of claim 8 in which said silica comprises an intermediate density silica gel or a modified regular density silica gel.

10. The process of claim 9 in which said silica gel has an apparent (bulk) density of about 0.35 to about 0.40 g/ml.

11. The process of claim 9 in which said silica has a surface area of about 300 to about 350 $m^2/g$.

12. The process of claim 9 in which said silica gel has an average pore diameter of about 60 to about 300 $\overset{\text{o}}{\text{A}}$.

13. The process of claim 12 in which said average pore diameter is about 120 to about 160 $\overset{\text{o}}{\text{A}}$.